# EUROPEAN PATENT SPECIFICATION

(11) **EP 1 594 536 B1**
(45) Date of publication and mention of the grant of the patent: **25.03.2009**
(21) Application number: 04706554.5
(22) Date of filing: 29.01.2004
(51) Int. Cl.: A61K 39/02, A61K 45/00, A61K 39/295, A61K 39/145

(54) **ADJUVANTED INFLUENZA VACCINE**
ADJUVANTE INFLUENZA-VAKZINE
VACCIN CONTRE LA GRIPPE CONTENANT UN ADJUVANT

(30) Priority: 30.01.2003 US 443985 P
(43) Date of publication of application: 16.11.2005
(73) Proprietor: Novartis Vaccines and Diagnostics, Inc., Emeryville, CA 94608 (US)
(72) Inventor: O'HAGAN, Derek, Emeryville, California 94662-8097 (US)
(74) Representative: Marshall, Cameron John
(86) International application number: PCT/US2004/002745
(87) International publication number: WO 2004/075829

(56) References cited:
- WO-A-99/27960
- WO-A2-03/028661
- US-A- 6 048 536
- US-A1- 2002 025 326
- US-A1- 2002 165 176
- US-B1- 6 391 318
- US-B1- 6 534 065
- SINGH M ET AL: "A NOVEL BIOADHESIVE INTRANASAL DELIVERY SYSTEM FOR INACTIVATED INFLUENZA VACCINES" JOURNAL OF CONTROLLED RELEASE, ELSEVIER, AMSTERDAM, NL, vol. 70, no. 3, 23 February 2001 (2001-02-23), pages 267-276, XP001100691 ISSN: 0168-3659
- ILLUM L ET AL: "CHITOSAN AS A NOVEL NASAL DELIVERY SYSTEM FOR VACCINES" ADVANCED DRUG DELIVERY REVIEWS, AMSTERDAM, NL, vol. 51, no. 1-3, 23 September 2001 (2001-09-23), pages 81-96, XP001115064 ISSN: 0169-409X
- BARACKMAN J D ET AL: "Intranasal immunization of mice with influenza vaccine in combination with the adjuvant LT-R72 induces potent mucosal and serum immunity which is stronger than that with traditional intramuscular immunization" INFECTION AND IMMUNITY, AMERICAN SOCIETY FOR MICROBIOLOGY. WASHINGTON, US, vol. 67, no. 8, August 1999 (1999-08), pages 4276-4279, XP002176322 ISSN: 0019-9567
- PINE S ET AL: "Intranasal immunization with influenza vaccine and a detoxified mutant of heat labile enterotoxin from Escherichia coli (LTK63)" JOURNAL OF CONTROLLED RELEASE, ELSEVIER, AMSTERDAM, NL, vol. 85, no. 1-3, 13 December 2002 (2002-12-13), pages 263-270, XP004397784 ISSN: 0168-3659
- BARCHFELD, G.L. ET AL.: 'The adjuvants MF59 and LT-K63 enhance the mucosal and systemic immunogenicity of subunit influenza vaccine administered intranasally in mice' VACCINE vol. 17, 1999, pages 695 - 704, XP004154808

## Description

### TECHNICAL FIELD

This invention is in the field of vaccines, particularly against influenza infection and disease.

### BACKGROUND

Inactivated influenza vaccines are widely available.. *See, e*.*g*. see Chapter 21 of *Vaccines*, eds. Plotkin & Orenstein, 3^{rd} edition (1999) ISBN 0-7216-7443-7. These are generally administered intramuscularly, although the vaccines are also immunogenic by the subcutaneous, intradermal, respiratory tract and oral routes.

An attractive route of administration for flu vaccines is the intranasal route, and this has received much attention. *See, e*.*g*., Gluck et al. (2002) J. Aerosol Med 15:221-228; U.S. Patent No. 6,534,065; Harper et al. (2003) MMWR Recomm Rep. 2003 Sep 26;52(RR-13):1-8. Singh et al (2001) J. Control Release 70(3): 267-276 discloses a vaccine composition comprising purified influenza hemagglutinin, LTK63 or LTR72 as adjuvants, and esterified hyaluronic acid (HYAFF) microspheres as a biospheres as a bioadhesive.

It is an object of the invention to provide modified and improved flu vaccines, and in particular to provide flu vaccines suitable for intranasal or other mucosal administration.

### DISCLOSURE OF THE INVENTION

The invention provides an immunogenic composition comprising: (a) an influenza antigen, (b) a detoxified ADP-ribosylating toxin, and (c) chitosan. The inclusion of chitosan allows a lower dose of the toxin to be used, thereby improving safety.

The composition is preferably suitable for mucosal administration *e*.*g*. intranasal administration.

### Influenza antigen

For vaccine production, influenza virus has traditionally been grown in embryonated hens eggs and purified by zonal centrifugation or chromatography. The virus can be included in vaccines as whole virions, but it is more common to disrupt the virus in order to decrease toxicity and reactogenicity. Treatment with detergent or organic solvent, for instance, yields "split" vaccines in which immunogenic surface glycoproteins are retained. Further purification gives "subunit" or purified surface antigen vaccines, which consist mainly of hemagglutinin (HA) and neuraminidase (NA).

Because of problems associated with retention of allergenic proteins, more modem production techniques have moved away from the use of eggs and towards cell culture, as recommended by the WHO in 1995. Typical cell lines for influenza culture include MDCK and Vero cell lines. To reduce contamination, cells are preferably grown in a serum-free or protein-free medium. To reduce contamination even further, and to reduce host cell DNA levels, virions can be treated by a process involving treatment with DNAse and cationic detergent. *See, e*.*g*., U.S. Patent No. 5,948,410.

Other modem approaches to influenza vaccination are reviewed in Palese et al. (2002) J. Clin Invest 110:9-13.

Any of these influenza antigens prepared in any of these ways may be used with the invention, but it is preferred (a) to use purified HA and, optionally, purified NA, and (b) to use antigens purified from cell lines rather than from eggs.

Whatever antigen(s) is/are used, it is preferred that it/they is/are selected to offer suitable coverage of existing strains. Updated guidelines on strains and subtypes are regularly issued by bodies such as the WHO, but in general it is preferred to include antigens from more than one strains, and containing a least one type A virus (e.g. A/H1N1 and A/H3N2) and at least one type B virus.

In one embodiment, the antigens are selected from a flu strain which is capable of or has the potential for causing a pandemic outbreak. Typically, a pandemic flu strain contains a haemagglutinin protein which is different from currently circulating strains or which has not be evident in the human population for an extended period of time. Examples of haemagglutinin proteins potentially associated with a pandemic flu strain include H2, H5, H6 or H9. Such antigens are discussed, for instance, in Hilleman, "Realities and enigmas of human viral influenza: pathogenesis, epidemiology and control", Vaccine (2002) 20:3068-3087 and Ha, et al., "X-ray structures of H5 avian and H9 swine influenza virus hemagglutinins bound to avian and human receptor analogs", PNAS (2001) 98(20): 11181-11186.

As an alternative to using glycoprotein antigens in the composition of the invention, nucleic acid encoding the antigen(s) may be used instead. *See, e*.*g*., Palese et al, *supra*; Ulmer et al. (2002) Vaccine 20 Suppl 2:S74-76. Protein components of the mixture may thus be replaced by nucleic acid (preferably DNA *e*.*g*. in the form of a plasmid) that encodes the protein.

### Detoxified ADP-ribosylating toxin

ADP-ribosylating bacterial exotoxins which catalyse the transfer of an ADP-ribose unit from NAD⁺ to a target protein are widely known. Examples include diphtheria toxin (*Corynebacterium diphtheriae*), exotoxin A (*Pseudomonas aeruginosa*), cholera toxin (CT; *Vibrio cholerae*), heat-labile enterotoxin (LT; *E. coli*) and pertussis toxin (PT). Further examples are disclosed in WO 02/079242 and *The Comprehensive Sourcebook of Bacterial Protein Toxins* (Alouf & Freer) ISBN 0120530759.

The toxins are typically divided into two functionally distinct domains - A and B. The A subunit is responsible for the toxic enzymatic activity, whereas the B subunit is responsible for cellular binding. The subunits might be domains on the same polypeptide chain, or might be separate polypeptide chains. The subunits may themselves be oligomers *e*.*g*. the A subunit of CT consists of A₁ and A₂ which are linked by a disulphide bond, and its B subunit is a homopentamer. Typically, initial contact with a target cell is mediated by the B subunit and then subunit A alone enters the cell.

The toxins are typically immunogenic, but their inclusion in vaccines is hampered by their toxicity. To remove toxicity without also removing immunogenicity, the toxins have been treated with chemicals such as glutaraldehyde or formaldehyde. A more rational approach relies on site-directed mutagenesis of key active site residues to remove toxic enzymatic activity whilst retaining immunogenicity (*see, e*.*g*. International Publication WO 93/1302 (CT and LT); European Patent Applications 0306618; 0322533; and 0322115 (PT), Del Giudice et al. (1999) Vaccine 17 Suppl. 2:S44-52). Current acellular whooping cough vaccines include a form of pertussis toxin with two amino acid substitutions (Arg⁹→Lys and Glu¹²⁹→Gly; 'PT-9K/129G'). *See, e.g.,* European Patent Application 0396964.

As well as their immunogenic properties, the toxins have been used as adjuvants. Parenteral adjuvanticity was first observed in (Northrup & Fauci (1972) J. Infect. Dis. 125:672ff) and mucosal adjuvanticity in 1984 (Elson & Ealding (1984) J. Immunol. 133:2392ff and 132:2736ff). It was surprisingly found in 1993 that the detoxified forms of the toxins retain adjuvanticity (International Publication WO 95/17211).

The compositions of the invention include a detoxified ADP-ribosylating toxin. The toxin may be diphtheria toxin, *Pseudomonas* exotoxin A or pertussis toxin, but is preferably cholera toxin (CT) or, more preferably, *E.coli* heat-labile enterotoxin (LT). Other toxins that can be used are those disclosed in WO 02/079242 (SEQ IDs 1 to 7 therein, and mutants thereof).

Detoxification of these toxins without loss of immunogenic and/or adjuvant activity can be achieved by any suitable means, with mutagenesis being preferred. Mutagenesis may involve one or more substitutions, deletions and/or insertions.

Preferred detoxified mutants are LT having a mutation at residue Arg-7 (*e*.*g*. a Lys substitution); CT having a mutation at residue Arg-7 (*e*.*g*. a Lys substitution); CT having a mutation at residue Arg-11 (*e*.*g*. a Lys substitution); LT having a mutation at Val-53; CT having a mutation at Val-53; CT having a mutation at residue Ser-61 (*e*.*g*. a Phe substitution); LT having a mutation at residue Ser-63 (*e*.*g*. a Lys or Tyr substitution as described in Chapter 5 of Del Giudice et al. (1998) Molecular Aspects of Medicine, vol. 19, number 1; Y63 described in Park et al (2000) Exp. Mol. Med. 32:72-78); CT having a mutation at residue Ser-63 (*e*.*g*. a Lys or Tyr substitution); LT having a mutation at residue Ala-72 (*e*.*g*. an Arg substitution as described in International Publication WO 98/18928); LT having a mutation at Val-97; CT having a mutation at Val-97; LT having a mutation at Tyr-104; CT having a mutation at Tyr-104; LT having a mutation at residue Pro-106 (*e*.*g*. a Ser substitution); CT having a mutation at residue Pro-106 (*e*.*g*. a Ser substitution); LT having a mutation at Glu-112 (*e*.*g*. a Lys substitution); CT having a mutation at Glu-112 (*e*.*g*. a Lys substitution); LT having a mutation at residue Arg-192 (*e*.*g*. a Gly substitution); PT having a mutation at residue Arg-9 (*e*.*g*. a Lys substitution); PT having a mutation at Glu-129 (*e*.*g*. a Gly substitution); and any of the mutants disclosed in International Publication WO 93/13202.

The amino acid sequences for CT and LT are described in Domenighini et al., Molecular Microbiology (1995) 15(6): 1165 -1167. This reference and the CT and LT sequence alignment disclosed therein are incorporated herein in their entirety.

These mutations may be combined *e*.*g*. Arg-9-Lys + Glu-129-Gly in PT, or LT with both a D53 and a K63 mutation, *etc*.

LT with a mutation at residue 63 or 72 is a preferred detoxified toxin. The LT-K63 and LT-R72 toxins are particularly preferred. *See, e*.*g*., Pizza et al. (2000) Int. J. Med. Microbiol. 290:455-461.

It will be appreciated that the numbering of these residues is based on prototype sequences and that, for example, although Ser-63 may not actually be the 63rd amino acid in a given LT variant, an alignment of amino acid sequences will reveal the location corresponding to Ser-63.

The detoxified toxins may be in the form of A and/or B subunits as appropriate for activity.

### Chitosan

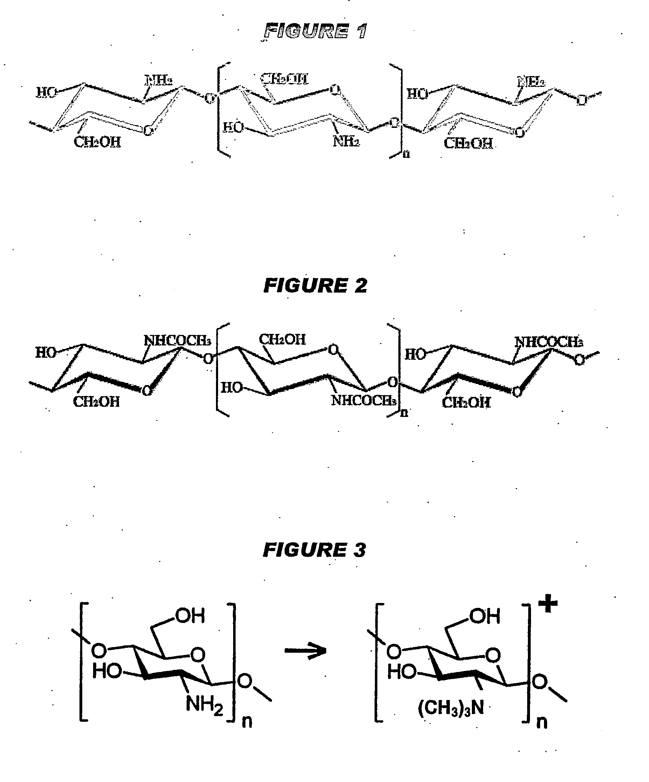

Chitosan has been reported as an adjuvant (*see, e*.*g*., U.S. Patent Nos. 6,534,065; ,912,000; 6,048,536; 6,136,606; 6,391,318; International Publication Nos. WO 96/09805, WO 96/10421, WO 97/01330; WO 97/16208, WO 97/20576; WO 98/42374; WO 99/27960; WO 01/35994; van der Lubben et al. (2001) Eur. J. Phann Sci. 14:201-207; Le Buanec et al. (2001) Biomed. Pharmacother. 55:316-320; Seferian & Martinez et al. (2000) Vaccine 19:661-668; Jabbal-Gill et al. (1998) Vaccine 19:2039-2046 Illum et al. (2001) Adv. Drug Deliv Rev 51 (1-3): 81-96; and Marcinkiewicz et al. (1991) Arch. Immunol. Ther. Exp. (Warsz) 39:127-132), particularly for mucosal (*e*.*g*. intranasal) use. Chitosan (Figure 1) is a N-deacetylated derivative of the exoskeletal polymer chitin (Figure 2), although the N-deacetylation is almost never complete. The deacetylation means that, unlike chitin, chitosan is soluble in dilute aqueous acetic and formic acids. Chitosan has also found wide applicability in non-vaccine pharmaceutical fields. *See*, *e*.*g*., Singla & Chawla (2001) J. Pharm. Pharmacol. 53:1047-1067.

The repeating glucosamine monomer of chitosan contains an amine group. This group may exist as free amine (-NH₂) or as cationic amine (-NH₃⁺), with protonation affecting the polymer's solubility. The amine groups are chemically active and can be substituted. Of particular interest for the invention, the amine groups can be substituted with one or more alkyl group ('A' *e*.*g*. methyl, ethyl, propyl, butyl, pentyl, *etc*.) *e*.*g*. -NHA, -NH₂A⁺, -NA¹A², -NHA¹A²⁺, NA¹A²A³⁺. Preferred derivatives are tri-alkylated and particularly preferred derivatives are trimethylated (*i*.*e*. trimethylchitosan, or 'TMC' - Figure 3). These derivatives much higher aqueous solubility than unmodified chitosan over a broader pH range.

It is not necessary for every amine in the chitosan polymer to be substituted in this way. The degree of substitution along the length of the chitosan chain can be determined by ¹H-NMR and can be controlled by means of the number and duration of reaction steps. *See, e.g.,* Hwang et al. (2002) J. Agric. Food Chem. 50:1876-1882. It is preferred that at least 10% (*e*.*g*. at least 20%, 30%, 40%, 50%, 60%, 70%, 80%, 90%, 95% or more) of monomers have a substituted amine.

There are two main reasons why it is rare that 100% of monomers in the chitosan will carry an alklyated amine. First, the substitution reaction will not usually be 100% efficient. Second, it is rare to find chitosan in which 100% of the monomer units carry amine groups because deacetylation of chitin is not usually 100% efficient. Alkylated chitosan derivatives used in the invention may therefore have amide and/or non-alkylated groups on some monomer units, and chitosan may possess some amide groups. Chitosan and derivatives used with the invention are preferably at least 75% deacetylated.

Chitosans come in a variety of molecular weights *e*.*g*. from oligosaccharides with molecular weight around 5,000-10,000 to polymers of high molecular weight (*e*.*g*. 600,000 - 1,000,000).

Where a cationic chitosan or derivative is used, it will be in the form of a salt *e*.*g*. chloride or lactate.

The chitosan or derivative can take various physical forms *e*.*g* in solution, as a powder, or in particulate form. Particulate forms are preferred, including microparticles, which may be cross-linked or non-cross-linked and may be formed conveniently by spray-drying. *See, e*.*g*., He et al. (1999) Int. J Pharm. 187:53-65; He et al. (1999) J Microencapsul. 16:343-355. Other physical forms include gels, beads, films, sponges, fibres, emulsions, *etc*.

The term "chitosan" as used with reference to the compositions, processes, methods and uses of the invention includes all these forms and derivatives of chitosan.

### Mucosal administration

Preferred compositions are suitable for mucosal delivery. Of the various mucosal delivery options available, the intranasal route is the most practical as it offers easy access with relatively simple devices that have already been mass-produced. Alternative routes for mucosal delivery of the composition are oral, intragastric, pulmonary, transdermal, intestinal, rectal, ocular, and vaginal routes.

### Preparation and presentation of compositions of the invention

The influenza antigen, detoxified ADP-ribosylating toxin and chitosan will be prepared separately and then admixed to give a composition of the invention. The composition can then be presented and packaged in various ways.

Where compositions are for injection, they may be presented in vials, or they may be presented in ready-filled syringes. The syringes may be supplied with or without needles. A syringe will include a single dose of the composition, whereas a vial may include a single dose or multiple doses. Injectable compositions will usually be liquid solutions or suspensions. Alternatively, they may be presented in solid form for solution or suspension in liquid vehicles prior to injection.

However, preferred compositions are for mucosal delivery, and the composition may thus be adapted for and/or packaged for mucosal administration. *See, e*.*g*., Almeida & Alpar (1996) J Drug Targeting 3:455-467; Agarwal & Mishra (1999) Indian J Exp Biol 37:6-16. Where the composition is for oral administration, for instance, it may be in the form of tablets or capsules (optionally enteric-coated), liquid, transgenic plants, drops, inhaler, aerosol, enteric coating, suppository, pessary, *etc*.. (*see, also* Michetti (1998) J. Gastroenterol SuppIX:66-68 and Chapter 17 of Vaccine design: the subunit and adjuvant approach, eds. Powell & Newman, Plenum Press 1995 (ISBN 0-306-44867). For the preferred mucosal route, the composition is preferably adapted for and/or packaged for intranasal administration, such as by nasal spray, nasal drops, gel or powder. *See, e*.*g*., Almeida & Alpar (1996) J Drug Targeting 3:455-467; Agarwal & Mishra (1999) Indian J Exp Biol 37:6-16.

Whatever the route of delivery, compositions of the invention are preferably packaged in unit dose form. Effective doses can be routinely established. A typical human dose of the composition for intranasal use has a volume of between 0.1 and 0.5ml *e*.*g*. two 100µl sprays, one per nostril.

Within each dose, the amount of individual antigens can be varied and tested by routine methods. For intranasal administration, however, HA can be administered at around 7.5µg per dose.

Compositions of the invention are preferably sterile. They are preferably pyrogen-free. They are preferably buffered *e*.*g*. at between pH 6 and pH 8, generally around pH 7. Where a composition comprises an aluminum hydroxide salt, it is preferred to use a histidine buffer. *See, e*.*g*., International Publication PCT/IB02/03495.

### Adjuvants

The toxin and chitosan act as mucosal adjuvants within the compositions of the invention. It is also possible to include one or more further mucosal adjuvants *e*.*g*.: (A) microparticles (*i*.*e*. a particle of ∼100nm to ∼150µm in diameter, more preferably ∼200nm to ∼30µm in diameter, and most preferably ∼500nm to ∼10µm in diameter) formed from materials that are biodegradable and non-toxic (*e*.*g*. a poly(α-hydroxy acid), a polyhydroxybutyric acid, a polyorthoester, a polyanhydride, a polycaprolactone *etc*., such as poly(lactide-co-glycolide) *etc*.) optionally treated to have a negatively-charged surface (*e*.*g*. with SDS) or a positively-charged surface (*e*.*g*. with a cationic detergent, such as CTAB); (B) monophosphoryl lipid A mimics, such as aminoalkyl glucosaminide phosphate derivatives *e*.*g*. RC-529 (Johnson et al. (1999) Bioorg Med Chem Lett 9:2273-2278); (C) polyphosphazene (PCPP); (D) a polyoxyethylene ether or a polyoxyethylene ester (International patent application WO 99/52549); (E) a polyoxyethylene sorbitan ester surfactant in combination with an octoxynol (International patent application WO 01/21207) or a polyoxyethylene alkyl ether or ester surfactant in combination with at least one additional non-ionic surfactant such as an octoxynol (International patent application WO 01/21152); (F) chitosan (International patent application W0 99/27960) ; (G) an immunostilnulatory oligonucleotide (*e*.*g*. a CpG oligonucleotide) and a saponin (International patent application WO 00/62800); and (H) liposomes (*see, e*.*g*., Chapters 13 & of Vaccine design: the subunit and adjuvant approach, eds. Powell & Newman, Plenum Press 1995 (ISBN 0-306-44867-X). Other mucosal adjuvants are also available (*see, e*.*g*. Chapter 7 of *Vaccine design: the subunit and adjuvant approach*, eds. Powell & Newman, Plenum Press 1995 (ISBN 0-306-44867-X)).

In addition to the mucosal adjuvants given above, the compositions of the invention may include one or more further adjuvants selected from the following group: (A) aluminum salts (alum), such as aluminum hydroxides (including oxyhydroxides), aluminum phosphates (including hydroxyphosphates), aluminum sulfate, *etc* (Chapters 8 & 9 of *Vaccine design: the subunit and adjuvant approach,* eds. Powell & Newman, Plenum Press 1995 (ISBN 0-306-44867-X)); (B) oil-in-water emulsion formulations (with or without other specific immunostimulating agents such as muramyl peptides [Muramyl peptides include N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE), *etc*.] or bacterial cell wall components), such as for example (a) MF59™ (Chapter 10 of *Vaccine design: the subunit and adjuvant approach,* eds. Powell & Newman, Plenum Press 1995 (ISBN 0-306-44867-X); International Publication W0 90/14837; US Patent No. 6,299,884) containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) Ribi^{™} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}); (C) saponin adjuvants (chapter 22 of *Vaccine design: the subunit and adjuvant approach,* eds. Powell & Newman, Plenum Press 1995 (ISBN 0-306-44867-X), such as QS21 or Stimulon^{™} (Cambridge Bioscience, Worcester, MA), either in simple form or in the form of particles generated therefrom such as ISCOMs (immunostimulating complexes; Chapter 23 of of *Vaccine design: the subunit and adjuvant approach*, eds. Powell & Newman, Plenum Press 1995 (ISBN 0-306-44867-X)), which ISCOMS may be devoid of additional detergent *e*.*g*. WO 00/07621; (D) Complete Freund's Adjuvant (CFA) and Incomplete Freund's Adjuvant (IFA); (E) cytokines, such as interleukins (e.g. IL-1, IL-2, IL-4, IL-5, IL-6, IL-7, IL-12, *etc*.) (International Publication W099/44636), interferons (*e*.*g*. gamma interferon), macrophage colony stimulating factor (M-CSF), tumor necrosis factor (TNF), *etc*.; (F) monophosphoryl lipid A (MPL) or 3-O-deacylated MPL (3dMPL) *e*.*g*. GB-2220221 and EP-A-0689454, optionally in the substantial absence of alum when used with pneumococcal saccharides *e*.*g*. International Publication WO 00/56358; (G) combinations of 3dMPL with, for example, QS21 and/or oil-in-water emulsions *e*.*g*. EP-A-0835318; EP-A-0735898; and EP-A-0761231; (H) oligonucleotides comprising CpG motifs *i*.*e*. containing at least one CG dinucleotide, with 5-methylcytosine optionally being used in place of cytosine; (I) an immunostimulant and a particle of metal salt *e.g.* WO 00/23105; (J) a saponin and an oil-in-water emulsion *e.g.* WO 99/11241; (K) a saponin (*e*.*g*. QS21) + 3dMPL + IL-12 (optionally + a sterol) *e*.*g*. WO 98/57659; (L) double-stranded RNA; (M) other substances that act as immunostimulating agents to enhance the effectiveness of the composition (*e*.*g*. chapter 7 of *Vaccine design: the subunit and adjuvant approach*, eds. Powell & Newman, Plenum Press 1995 (ISBN 0-306-44867-X)). With certain adjuvants, for example aluminum salts, the flu antigen(s) may be adsorbed to the aluminum salt.

### Further components of the compositions

The compositions of the invention include an influenza antigen. However, the invention can also be applied to other antigens, instead of or in addition to influenza antigens. The composition of the invention may thus include one or more of the following antigens, in place of or in addition to the influenza antigen(s) described above:
- antigens from *Helicobacter pylori* such as CagA (*see, e*.*g*., Covacci & Rappuoli (2000) J Exp. Med. 19:5 87-592; W093/18150; Covacci et al. (1993) Proc. Natl. Acad. Sci. USA 90: 5791-5795; Tummuru et al. (1994) Infect. Immun. 61:1799-1809); VacA (*see, e*.*g*., Marchetti et al. (1998) Vaccine 16:33-37; Telford et al (1994) J Exp. Med. 179:1653-1658); NAP (*see, e*.*g*., Evans et al (1995) Gene 153:123-127; International Publications W0 96/01272; WO 96/01273 (esp. SEQ ID NO:6); W097/25429), HopX (*see, e*.*g*., International Publication W0 98/04702), HopY (*see, e*.*g*., International Publication W0 98/04702) and/or urease.
- a saccharide antigen from *Streptococcus pneumoniae* (*see, e*.*g*., Watson (2000) Pediatr. Infect. Dis J 19:331-332; Rubin (2000) Pediatr Clin North Am 47:269-285; Jedrzejas (2001) MicrohiolMolBiolRev 65:187-207).
- an antigen from hepatitis A virus, such as inactivated virus (*see, e*.*g*., Bell (2000) Pediatr Infect Dis J 19:1187-1188; Iwarson (1995) APMIS 103:321-326)
- an antigen from hepatitis B virus, such as the surface and/or core antigens (*see, e*.*g*., Gerlich et al. (1990) Vaccine 8 Suppl:S63-68 & 79-80), with surface antigen preferably being adsorbed onto an aluminum phosphate *(see, e.g.,* International Publication W093/24148).
- a protein from serogroup B of *N. meningitidis* (*see, e*.*g*., International Publications W0 99/24578, W0 99/36544; W099/57280; WO 00/22430; Tettelin et al. (2000) Science 287:1809-1815; Pizza et al (2000) Science 287:1816-1820).
- an OMV preparation from serogroup B of *N. meningitidis* (*see, e*.*g*., International Publication WO 01/52885; Bjune et al. (1991) Lancet 338(8775):1093-1096; Fukasawa et al. (1999) Vaccine 17:2951-2958; Rosenqvist et aL (1998) Dev. Biol. Stand. 92:323-333).
- a saccharide antigen from *Hemophilus influenzae* B, preferably non-adsorbed or adsorbed onto an aluminum phosphate (*see, e*.*g*., International Publication W0 97/00697).
- an antigen from hepatitis C virus (*see, e.g.,* Hsu et al. (1999) Clin Liver Dis 3:901-915*).*
- an antigen from *N. gonorrhoeae* (*see, e*.*g*., International Publications W0 99/24578, W0 99/36544; W099/57280; WO 00/22430).
- an antigen from *Chlamydia pneumoniae* (*see, e.g.,* International Publications W0 02/02606; WO 00/27994; W0 99/27105; WO 00/37494; Kalman et al. (1999) Nature Genetics 21:385-389; Read et al. (2000) Nucleic Acids Res 28:1397-406; Shirai et al. (2000) J Infect. Dis. 181(Suppl 3):S524-S527).
- an antigen from *Chlamydia trachomatis* (*see, e*.*g*., W0 99/28475).
- an antigen from *Porphyromonas gingivalis* (*see, e*.*g*., Ross et al. (2001) Vaccine 19:41354142).
- polio antigen(s) (*see, e*.*g*., Sutter et al. (2000) Pediatr Clin North Am 47:287-308; Zimmerman & Spann (1999) Am Fam Physician 59:113-118,125-126) such as IPV.
- rabies antigen(s) (*see, e*.*g*., Dreesen (1997) Vaccine 15 Suppl:S2-6) such as lyophilised inactivated virus, RabAvert™ as described in MMWR Morb Mortal Wkly Rep 1998 Jan 16;47(1):12,19].
- measles, mumps and/or rubella antigens (*see, e*.*g*. Chapters 12, 13 & 17 of Vaccines, eds. Plotkin & Orenstein, 3rd edition (1999) ISBN 0-7216-7443-7)
- an antigen from *Moraxella catarrhalis* (*see, e*.*g*., McMichael (2000) Vaccine 19 Suppl 1: S101-107).
- an antigen from *Streptococcus agalactiae* (group B streptococcus) (*see, e*.*g*., Schuchat (1 999) Lancet 353(9146):51-6; International Publication W002/34771).
- an antigen from *Streptococcus pyogenes* (group A streptococcus) (*see, e*.*g*., International Publication W0 02/34771; Dale (1999) Infect Dis Clin North Am 13:227-43, viii; Ferretti et al. (2001) PNAS USA 98: 4658-4663).
- an antigen from *Staphylococcus aureus* (*see, e*.*g*., Kuroda et al (2001) Lancet 357(9264):1225-1240; see also pages 1218-1219).
- antigen(s) from a paramyxovirus such as respiratory syncytial virus (RSV, *see, e*.*g*., Anderson (2000) Vaccine 19 Suppl 1:S59-65; Kahn (2000) Curr Opin Pediatr 12:257-262) and/or parainfluenza virus (PIV3, *see, e*.*g*,. Crowe (1995) Vaccine 13:415-421*).*
- an antigen from *Bacillus anthracis* (*see, e*.*g*., J Toxicol Clin Toxicol (2001) 39:85-100; Demicheli et al. (1998) Vaccine 16:880-884; Stepanov et al. (1996) J Biotechnol 44:155-160).
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen *e*.*g*. from parvovirus B19.
- a tetanus toxoid (*see, e*.*g*. chapter 18 of Vaccines, eds. Plotkin & Orenstein, 3rd edition (1999) ISBN 0-7216-7443-7)
- pertussis holotoxin (PT) and filamentous hemagglutinin (FHA) from B.pertussis, optionally also in combination with pertactin and/or agglutinogens 2 and 3 (*see, e*.*g*., Gustafasson et al. (1996) N. Engl. J Med. 334:349-355; Rappuoli et al (1991) TIBTECH 9:232-238).
- cellular pertussis antigen.

The mixture may comprise one or more of these further antigens, which may be detoxified where necessary (*e*.*g*. detoxification of pertussis toxin by chemical and/or genetic means).

Where a diphtheria antigen is included in the mixture it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens in the mixture will typically be present at a concentration of at least 1µg/ml each. In general, the concentration of any given antigen will be sufficient to elicit an immune response against that antigen.

As an alternative to using proteins antigens in the mixture, nucleic acid encoding the antigen may be used. Protein components of the mixture may thus be replaced by nucleic acid (preferably DNA *e*.*g*. in the form of a plasmid) that encodes the protein. Similarly, compositions of the invention may comprise proteins which mimic saccharide antigens *e*.*g*. mimotopes (*see, e*.*g*., Charalambous & Feavers (2001) J Med Microbiol 50:937-939) or anti-idiotype antibodies. These may replace individual saccharine components, or may supplement them.

### Immunogenicity

Compositions of the invention are immunogenic. Preferred immunogenic compositions are vaccines. Vaccines according to the invention may either be prophylactic (*i*.*e*. to prevent infection) or therapeutic (*i*.*e*. to treat disease after infection), but will typically be prophylactic.

Immunogenic compositions and vaccines of the invention will, in addition to components described above, typically comprise 'pharmaceutically acceptable carriers', which include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, trehalose (*see, e*.*g*., International Publication WO 00/56365), lipid aggregates (such as oil droplets or liposomes), and inactive virus particles. Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. A thorough discussion of pharmaceutically acceptable excipients is available in Gennaro (2000) Remington. The Science and Practice of Pharmacy. 20th ed ISBN: 0683306472.

Immunogenic compositions used as vaccines comprise an immunologically effective amount of influenza antigen, as well as any other of the above-mentioned components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e*.*g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

Methods for determining immunogenicity of influenza vaccines are well known.

### Administration of compositions of the invention

As mentioned above, compositions of the invention may be administered by various routes, including parenteral and mucosal. A preferred route of parenteral administration is injection. Injection may be subcutaneous, intraperitoneal, intravenous or intramuscular. Intramuscular administration to the thigh is preferred. Needle-free injection may be used. A preferred route of mucosal administration is intranasal. Transdermal or transcutaneous administration is also possible (*see, e*.*g*. W0 98/20734).

Administration may be a single dose schedule or a multiple dose schedule. A primary dose schedule may be followed by a booster dose schedule. Suitable timing between priming and boosting can be routinely determined.

Administration will generally be to an animal and, in particular, human subjects can be treated. The compositions are useful for vaccinating children and adults.

### Medical methods and uses

Compositions of the invention can be used in method of raising an immune response in a patient, comprising administering to a patient a composition of the invention. The immune response is preferably protective against influenza infection, and may comprise a humoral immune response and/or a cellular immune response.

The method may raise a booster response, in a patient that has already been primed against flu virus.

The invention also provides the use of (a) an influenza antigen, (b) a detoxified ADP-ribosylating toxin, and (c) chitosan, in the manufacture of a medicament for preventing influenza virus infection.

### Definitions

The term "comprising" means "including" as well as "consisting" *e*.*g*. a composition "comprising" X may consist exclusively of X or may include something additional *e*.*g*. X + Y.

The term "about" in relation to a numerical value x means, for example, *x*±10%.

The word "substantially" does not exclude "completely" *e*.*g*. a composition which is "substantially free" from Y may be completely free from Y. Where necessary, the word "substantially" may be omitted from the definition of the invention.

### BRIEF DESCRIPTION OF DRAWINGS

Figures 1 to 3 show the repeating structures of (1) chitosan (2) chitin and (3) trimethylchitosan.
**Figure 4** shows IgG titers after immunisation with HA-containing compositions, and Figure 5 shows HI titers from the same mice.

### MODES FOR CARRYING OUT THE INVENTION

Nine groups of mice (10 mice to a group) were given two 10µg intranasal doses at 4 week intervals of influenza virus hemagglutinin either alone, with a detoxified LT adjuvant, or with a detoxified LT adjuvant and 0.5% chitosan:

| Group | Adjuvant |
|---|---|
| 1 | None |
| 2 | LTK63, 1µg |
| 3 | LTK63, 10µg |
| 4 | LTR72, 1µg |
| 5 | LTR72, 10µg |
| 6 | Chitosan + LTK63, 1µg |
| 7 | Chitosan + LTK63, 10µg |
| 8 | Chitosan + LTR72, 1µg |
| 9 | Chitosan + LTR72, 10µg |

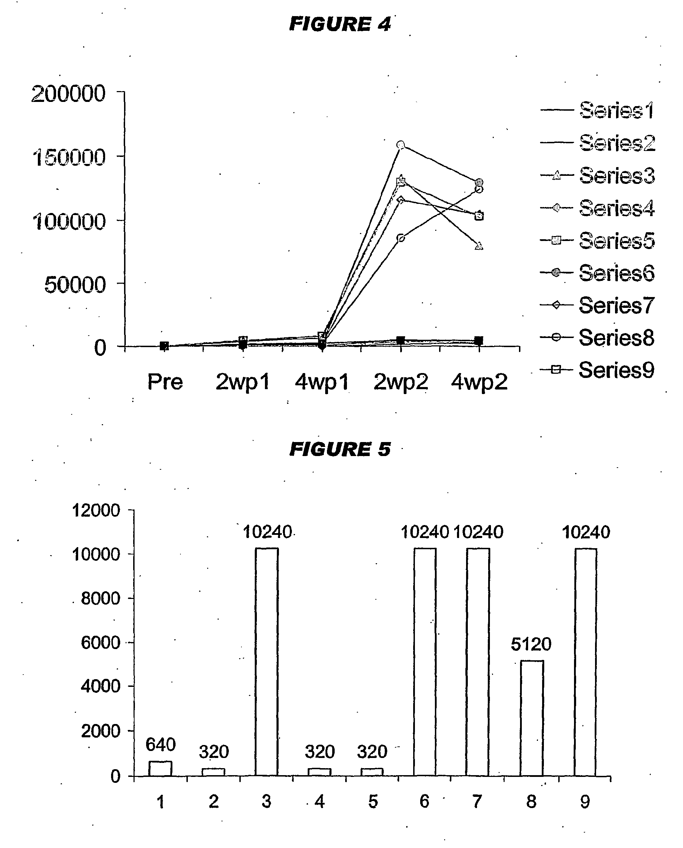

IgG titers were measured before immunisation, and at 2 and 4 weeks after both doses. Results are shown in Figure 4. Hemagglutination inhibition (HI) titers were measured 2 weeks after the second dose, and results are shown in Figure 5.

A comparison of groups 2 & 6 (1µg LTK63) shows that the addition of chitosan to LTK63 gives substantially better titers. The titers are increased to match those achieved with 10µg LTK63, and thus chitosan allows a lower dose of the toxin to be used.

## Claims

1. A composition comprising
an influenza antigen;
a detoxified ADP-ribosylating protein; and
a chitosan.

2. The composition of claim 1, wherein the influenza antigen comprises one or more surface antigens.

3. The composition of claim 2, wherein the surface antigens are haemagglutinin and/or neuraminidase.

4. The composition of claim 1, wherein the detoxified ADP-ribosylating protein is selected from the group consisting a detoxified diphtheria toxin protein, a detoxified exotoxin A protein, a detoxified cholera toxin (CT) protein; a detoxified heat-labile enterotoxin (LT) toxin protein; and a detoxified pertussis toxin (PT) protein.

5. The composition of claim 4, wherein the ADP-ribosylating protein is a detoxified LT, CT or PT protein.

6. The composition of claim 5, wherein the detoxified LT protein is selected from the group consisting of LTK7, LTX53, LTK63, LTY63, LTR72, LTX97, LTX104, LTS106, LTK112, LTG192, CTK7, CTK11, CTX53, CTF61, CTK63, CTY63, CTX97, CTX104, CTK112, CTS106, PTK9, PTG129 and combinations thereof.

7. The composition of any of the preceding claims, wherein the chitosan is at least 75% deacetylated.

8. The composition of claim 7, wherein the chitosan is alkylated.

9. The composition of claim 8, wherein the chitosan is trialkylated.

10. The composition of any of the preceding claims, wherein the influenza antigen is provided as a polynucleotide encoding the antigen.

11. The composition of any of the preceding claims, further comprising one or more additional antigens and/or one or more additional adjuvants.

12. The composition of any of the preceding claims which is adapted for mucosal administration.

13. The composition of claim 12, wherein the mucosal administration is intranasal.

14. The composition of claim 13, in the form a nasal spray or nasal drops.

15. A dispensing device in combination of the composition of any of the preceding claims, wherein the dispensing device is adapted to deliver the composition intranasally.

16. A kit comprising any of the compositions according to claims 1-14, wherein when combined or reconstituted, the composition is suitable for mucosal administration.

17. Use of the composition according to any of claims 1-14 in the manufacture of a medicament for generating an immune response to influenza in a subject.

18. The use of claim 17, wherein the immune response protects the subject against influenza infection or disease.

19. Use of at least one influenza antigen, a chitosan and a detoxified ADP-ribosylating protein in the manufacture of a medicament for mucosal delivery to an animal in order to raise an immune response.

20. Use of claim 19, wherein the medicament is for intranasal delivery.

## Patentansprüche

1. Zusammensetzung, umfassend
ein Influenza-Antigen;
ein detoxifiziertes ADP-ribosylierendes Protein; und
ein Chitosan.

2. Zusammensetzung nach Anspruch 1, wobei das Influenza-Antigen ein oder mehrere Oberflächen-Antigene umfasst.

3. Zusammensetzung nach Anspruch 2, wobei die Oberflächen-Antigene Hämagglutinin und/oder Neuraminidase sind.

4. Zusammensetzung nach Anspruch 1, wobei das detoxifizierte ADP-ribosylierende Protein ausgewählt ist aus der Gruppe bestehend aus einem detoxifizierten Diphtherie-ToxinProtein, einem detoxifizierten Exotoxin A-Protein, einem detoxifizierten Cholera-Toxin (CT)-Protein; einem detoxifizierten hitzelabilen Enterotoxin (LT)-Toxin-Protein; und einem detoxifizierten Pertussis-Toxin (PT)-Protein.

5. Zusammensetzung nach Anspruch 4, wobei das ADP-ribosylierende Protein ein detoxifiziertes LT-, CT- oder PT-Protein ist.

6. Zusammensetzung nach Anspruch 5, wobei das detoxifizierte LT-Protein ausgewählt ist aus der Gruppe bestehend aus LTK7, LTX53, LTK63, LTY63, LTR72, LTX97, LTX104, LTS106, LTK112, LTG192, CTK7, CTK11, CTX53, CTF61, CTK63, CTY63, CTX97, CTX104, CTK112, CTS106, PTK9, PTG129 und Kombinationen derselben.

7. Zusammensetzung nach einem der vorhergehenden Ansprüche, wobei das Chitosan zu mindestens 75 % deacetyliert ist.

8. Zusammensetzung nach Anspruch 7, wobei das Chitosan alkyliert ist.

9. Zusammensetzung nach Anspruch 8, wobei das Chitosan trialkyliert ist.

10. Zusammensetzung nach einem der vorhergehenden Ansprüche, wobei das Influenza-Antigen als Polynukleotid bereitgestellt wird, das für das Antigen kodiert.

11. Zusammensetzung nach einem der vorhergehenden Ansprüche, die ferner ein oder mehrere zusätzliche Antigene und/oder ein oder mehrere zusätzliche Adjuvantien umfasst.

12. Zusammensetzung nach einem der vorhergehenden Ansprüche, die für die mukosale Verabreichung angepasst ist.

13. Zusammensetzung nach Anspruch 12, wobei die mukosale Verabreichung intranasal erfolgt.

14. Zusammensetzung nach Anspruch 13, in Form eines Nasensprays oder in Form von Nasentropfen.

15. Verabreichungsvorrichtung in Kombination mit der Zusammensetzung nach einem der vorhergehenden Ansprüche, wobei die Verabreichungsvorrichtung für die intranasale Zuführung der Zusammensetzung angepaßt ist.

16. Kit, das eine Zusammensetzung nach einem der Ansprüche 1 bis 14 umfasst, wobei die Zusammensetzung für die mukosale Verabreichung geeignet ist, wenn sie kombiniert oder rekonstituiert wird.

17. Verwendung der Zusammensetzung nach einem der Ansprüche 1 bis 14 bei der Herstellung eines Medikamentes zur Erzeugung einer Immunantwort gegen Influenza in einem Individuum.

18. Verwendung nach Anspruch 17, wobei die Immunantwort das Individuum gegen eine Influenza-Infektion oder eine Influenza-Erkrankung schützt.

19. Verwendung mindestens eines Influenza-Antigens, eines Chitosans und eines detoxifizierten ADP-ribosylierenden Proteins bei der Herstellung eines Medikamentes für die mucosale Zuführung an ein Tier zur Erzeugung einer Immunantwort.

20. Verwendung nach Anspruch 19, wobei das Medikament für die intranasale Zuführung ist.

## Revendications

1. Composition comprenant
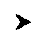 un antigène de la grippe ;
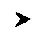 une protéine ADP-ribosylante détoxifiée ; et
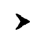 un chitosane.

2. Composition selon la revendication 1, dans laquelle l'antigène de la grippe comprend un ou plusieurs antigènes de surface.

3. Composition selon la revendication 2, dans laquelle les antigènes de surface sont l'hémagglutinine et/ou la neuraminidase.

4. Composition selon la revendication 1, dans laquelle la protéine ADP-ribosylante détoxifiée est choisie dans le groupe constitué d'une protéine de toxine diphtérique détoxifiée, une protéine d'exotoxine A détoxifiée, une protéine de toxine cholérique (CT) détoxifiée, une protéine d'entérotoxine thermolabile (LT) détoxifiée et une protéine de toxine pertussique (PT) détoxifiée.

5. Composition selon la revendication 4, dans laquelle la protéine ADP-ribosylante est une protéine LT, CT ou PT détoxifiée.

6. Composition selon la revendication 5, dans laquelle la protéine LT détoxifiée est choisie dans le groupe constitué de LTK7, LTX53, LTK63, LTY63, LTR72, LTX97, LTX104, LTS106, LTK112, LTG192, CTK7, CTK11, CTX53, CTF61, CTK63, CTY63, CTX97, CTX104, CTK112, CTS106, PTK9, PTG129 et des combinaisons de celles-ci.

7. Composition selon l'une quelconque des revendications précédentes, dans laquelle le chitosane est désacétylé à au moins 75 %.

8. Composition selon la revendication 7, dans laquelle le chitosane est alkylé.

9. Composition selon la revendication 8, dans laquelle le chitosane est trialkylé.

10. Composition selon l'une quelconque des revendications précédentes, dans laquelle l'antigène de la grippe est fourni sous la forme d'un polynucléotide codant pour l'antigène.

11. Composition selon l'une quelconque des revendications précédentes, comprenant en outre un ou plusieurs antigènes supplémentaires et/ou un ou plusieurs adjuvants supplémentaires.

12. Composition selon l'une quelconque des revendications précédentes, qui est adaptée pour une administration mucosale.

13. Composition selon la revendication 12, dans laquelle l'administration mucosale est intranasale.

14. Composition selon la revendication 13, sous la forme de pulvérisations nasales ou de gouttes nasales.

15. Dispositif de distribution en combinaison de la composition selon l'une quelconque des revendications précédentes, où le dispositif de distribution est adapté pour délivrer la composition par voie intranasale.

16. Kit comprenant l'une quelconque des compositions selon les revendications 1 à 14, dans lequel, lorsqu'elle est combinée ou reconstituée, la composition est appropriée pour une administration mucosale.

17. Utilisation de la composition selon l'une quelconque des revendications 1 à 14 dans la fabrication d'un médicament destiné à générer une réponse immunitaire contre la grippe chez un sujet.

18. Utilisation selon la revendication 17, où la réponse immunitaire protège le sujet contre une infection ou une maladie grippale.

19. Utilisation d'au moins un antigène de la grippe, un chitosane et une protéine ADP-ribosylante détoxifiée dans la fabrication d'un médicament pour une délivrance mucosale à un animal afin de soulever une réponse immunitaire.

20. Utilisation selon la revendication 19, où le médicament est pour une délivrance intranasale.
